# EUROPÄISCHE PATENTSCHRIFT

(11) **EP 1 458 418 B1**
(45) Veröffentlichungstag und Bekanntmachung des Hinweises auf die Patenterteilung: **19.04.2006**
(21) Anmeldenummer: 02795188.8
(22) Anmeldetag: 13.12.2002
(51) Int. Cl.: A61L 2/00, A61L 2/16, A61L 2/18, A61L 2/26, A01N 33/04, A01N 33/12, A01N 37/44, A01N 37/20, A61C 19/00

(54) **DESINFEKTIONSMITTEL FÜR ABSAUGANLAGEN IM MEDIZINISCHEN BEREICH**
DISINFECTION AGENT FOR SUCTION SYSTEMS USED IN THE FIELD OF MEDICINE
AGENT DESINFECTANT POUR INSTALLATIONS D'ASPIRATION DANS LE DOMAINE MEDICAL

(30) Priorität: 22.12.2001 DE 10163845
(43) Veröffentlichungstag der Anmeldung: 22.09.2004
(73) Patentinhaber: ECOLAB INC., St. Paul, MN 55102-1390 (US)
(72) Erfinder: BIERING, Holger, 41516 Grevenbroich (DE); VON RHEINBABEN, Friedrich, 40789 Monheim (DE); DECKER, Michael, 42697 Solingen (DE)
(74) Vertreter: Sternagel, Fleischer, Godemeyer & Partner
(86) Internationale Anmeldenummer: PCT/EP2002/014222
(87) Internationale Veröffentlichungsnummer: WO 2003/055532

(56) Entgegenhaltungen:
- DE-A- 4 007 758
- DE-A- 19 603 977
- DE-A- 19 613 881
- DE-U- 20 017 213
- US-B1- 6 331 607

## Beschreibung

Die vorliegende Erfindung liegt auf dem Gebiet der Desinfektion für Absauganlagen im medizinischen Bereich. Sie betrifft Desinfektionsmittel für ärztliche oder zahnärztliche Absauganlagen sowie als weiteren Gegenstand die Verfahren zur Pflege entsprechender Anlagen und die Verwendung eines derartigen Desinfektionsmittels zur Abtötung von Bakterien und/oder Pilzen in entsprechenden Absauganlagen.

Insbesondere im medizinischen Sektor ist es erforderlich, alle sinnvollen Maßnahmen zu treffen, die sowohl die Sicherheit der Patienten durch Verhinderung einer möglichen Übertragung von pathogenen bzw. fakultativ pathogenen Keimen zu gewährleisten als auch eine Belästigung durch geruchsbildende Bakterien zu vermeiden.

Ein möglicher Wachstumsherd für derartige Keime sind Absauganlagen im medizinischen Bereich, die deshalb regelmäßig gereinigt und desinfiziert werden müssen. Beispielsweise wird mit zahnärztlichen Absauganlagen ein Gemischstrom aus Luft, Wasser, Speichel, Zahnsubstanz und dergleichen aus dem Patientenmund angesaugt, von der Luft getrennt und der Rest einem Abfluß zugeführt. In den Saugschläuchen und Rohrleitungen setzen sich Bestandteile des Gemischstromes, insbesondere auch Bakterien, Blutreste und weitere Verschmutzungen ab. Durch regelmäßiges Spülen oder anderweitige Behandlung mit Desinfektionsmittel-Wasser-Gemischen versucht man, den hygienischen Anforderungen zu genügen. Zum Zwecke der Desinfektion sind bisher üblicherweise flüssige Desinfektionsmittel-Konzentrate auf der Basis von quartären Ammoniumverbindungen zum Einsatz gekommen, welche vor der Anwendung verdünnt werden. Der Nachteil dieses Verfahrens besteht in einer unzureichenden Verweildauer der Desinfektionslösung in der Absauganlage, da sich strömungstechnisch im Absaugschlauch Rinnsale bilden, wodurch keine gleichmäßige Benetzung der inneren Oberflächen der Schläuche gegeben ist. Des weiteren besitzen die eingesetzten quartären Ammoniumverbindungen nur ein begrenztes Wirkungsspektrum gegen Bakterien, Pilze und behüllte Viren. Insbesondere die Wirkung gegenüber Mycobakterien ist mit diesen Wirkstoffen nicht gewährleistet.

Eine weitere Anwendungsmöglichkeit des Stands der Technik stellt die Verwendung von pulverförmigen oder granulierten Desinfektionsmitteln gemäß der DE 40 10 615 dar. Erfolgt die Anwendung nach vorheriger Befeuchtung des Absaugsystems, so ist eine längere Verweildauer sowie eine ausreichende gleichmäßige Benetzung bei dem Verfahren gemäß der DE 40 10 615 möglich. Der Nachteil dieses Verfahrens besteht jedoch in der Gewährleistung einer homogenen Verteilung des Desinfektionswirkstoffes im eingesetzten Produktgemisch, welches weitere feste Rezepturbestandteile, wie beispielsweise Builder, Reinigungskomponenten und weitere Inhaltsstoffe enthält. Die geforderte Homogenität und Stabilität der Pulvermischung bzw. des Granulates ist nur durch technisch aufwendige Verfahren erreichbar. Ein Nachteil besteht auch in der Begrenzung der Auswahl von möglichen mikrobiziden Wirkstoffen, da vorzugsweise feste Substanzen, wie beispielsweise quartäre Ammoniumverbindungen oder Salze der Chlorisocyanursäure bzw. des Tosylchloramids zum Einsatz kommen können. Die Nachteile der Verwendung von quartären Ammoniumverbindungen wurden bereits beschrieben. Die chlorhaltigen Wirkstoffe sind demgegenüber AOX-bildende Substanzen, deren Einleitung in kommunale Abwassersysteme begrenzt ist. Unter AOX-bildenden Substanzen wird gemäß vorliegender Erfindung verstanden, daß diese Verbindungen zur Bildung von an Aktivchlor absorbierbaren organischen Halogenverbindungen führen können.

DE-A-196 03 977 beschreibt ein Verfahren zur Reinigung und Desinfektion von empfindlichen medizinischen Geräten, inbesondere von Endoskopen. Als Desinfektionsmittel wird eine wässrige Zusammensetzung aus Alkylpropylendiamin und Glucoprotamin eingesetzt.

Dementsprechend stellte sich die vorliegende Erfindung die Aufgabe, ein Desinfektionsmittel für Absauganlagen zur Verfügung zu stellen, welches eine gleichmäßige Verteilung der Wirksubstanzen in der Absauganlage gewährleistet und eine ausreichende Verweildauer für die Abtötung bzw. Inaktivierung eines umfas-sendenKeimspektrums, insbesondere von Mycobakterien und Adenoviren sicherstellt.

Gegenstand der vorliegenden Erfindung ist demzufolge ein verdicktes anwendungsfertiges Desinfektionsmittel für ärztliche oder zahnärztliche Absauganlagen, enthaltend einen Wirkstoff ausgewählt aus der Gruppe der Alkylpropylendiamine mit der allgemeinen Formel I 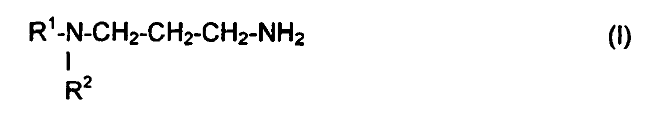 worin R¹ eine Alkyl- oder Alkenylgruppe mit 8 bis 18 Kohlenstoffatomen und R² Wasserstoff, eine Alkylgruppe mit 1 bis 4 Kohlenstoffatomen oder eine Aminoalkylgruppe mit 2 bis 4 Kohlenstoffatomen bedeuten und/oder der Gruppe der als Glucoprotamin® bekannten Produkte, wie sie aus Alkylpropylendiamin der Formel II

R³-NH-CH₂-CH₂-CH₂-NH₂ (II)

in der R³ für eine lineare Alkylgruppe mit 12 bis 14 Kohlenstoffatomen steht, durch Umsetzung mit Verbindungen der Formel III 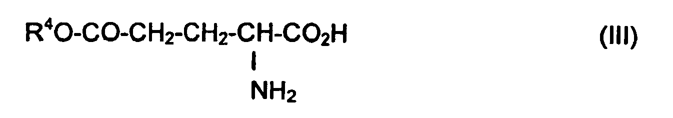 in der R⁴ für Wasserstoff oder eine Akylgruppe mit 1 bis 4 Kohlenstoffatomen steht, im Molverhältnis 1 : 1 bis 1 : 2 bei 60 bis 175°C zugänglich sind, dadurch gekennzeichnet, daß das anwendungsfertige Mittel eine Viskosität zwischen 100 und 2000 mPas, vorzugsweise zwischen 350 und 1000 mPas aufweist, gemessen mit einem Brookfield-Digitalviskosimeter, Modell LVTDV-II bei einer Probentemperatur von 20°C unter Verwendung der Spindel Nr. 2 (LV-Serie Codierzahl 62) mit einer Spindeldrehung von 12 Umdrehungen/Minute, wobei der Wert nach 60 Sekunden abgelesen wurde wobei als zusätcliche Komponenten Verdicket enthalten ist. Dabei bedeutet anwendungsfertig, daß das Mittel, das unmittelbar in die Absauganlage eingebracht wird, die genannten Eigenschaften hat.

Das erfindungsgemäße Desinfektionsmittel enthält vorzugsweise als genannten Wirkstoff Glucoprotamin®, N,N-Bis(3-aminopropyl)-laurylamin, N-Dodecyl-1,3-propandiamin, N-Kokos-1,3-propandiamin oder ein Gemisch hieraus.

Vorzugsweise sind in dem erfindungsgemäßen Desinfektionsmittel, bezogen auf das gesamte Mittel, 0,01 bis 30 Gew.-%, insbesondere 0,1 bis 15 Gew.-% von einem oder mehreren der genannten Wirkstoffe enthalten.

In einer weiteren bevorzugten Ausführungsform enthält das erfindungsgemäße Desinfektionsmittel zusätzlich einen zweiten Wirkstoff ausgewählt aus der Gruppe der quartären Ammoniumverbindungen der Formel I 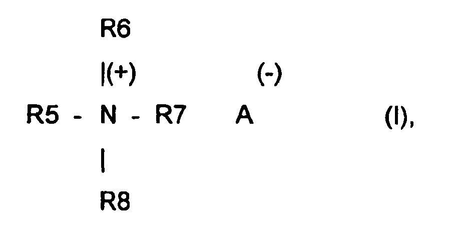 in der R5 eine Alkylgruppe mit 6 - 16 C-Atomen, R6 eine Alkylgruppe mit 1 - 12 C-Atomen oder eine Benzylgruppe, R7 und R8 Alkylgruppen mit 1 - 4 C-Atomen oder Hydroxyalkylgruppen mit 2 - 4 C-Atomen und A(-) die äquivalente Menge eines korrespondierenden Anions darstellt, enthalten ist.

Besonders bevorzugte Wirkstoffe gemäß Formel I sind dabei Benzalkoniumchlorid und/oder Dimethyl-dioctylammoniumchlorid.

Dabei ist es weiterhin bevorzugt, daß in dem erfindungsgemäßen Desinfektionsmittel, bezogen auf das gesamte Mittel, von 0,01 bis 30 Gew.-%, insbesondere 0,1 bis 15 Gew.-% des genannten zweiten Wirkstoffes enthalten sind.

Der Vorteil des vorliegenden erfindungsgemäßen Desinfektionsmittels ist, daß es nach Anwendung auf der Innenfläche des Saugschlauches oder der Rohre eine zusammenhängende Schicht bildet, wobei diese Schicht während einer bestimmten Zeitdauer, die abhängig ist von der Viskosität des Desinfektionsmittels im wesentlichen nicht völlig zerfließt. Ein flüssig vorliegendes Desinfektionsmittel ist deshalb keine Zusammensetzung im Sinne der vorliegenden Erfindung, da eine solche Lösung nach Einbringen in die Absauganlage fast augenblicklich zerfliesst.

Voraussetzung ist auch, daß das erfindungsgemäße Desinfektionsmittel nach dem Auftragen und Einwirken wieder abgewaschen werden kann.

Im Prinzip kann jedes beliebige Gel aus Dispersum und Dispergiermittel als Grundlage der Zubereitung dienen, sofern die Bestandteile nicht unerwünscht mit den erfindungsgemäßen Wirkstoff-Komponenten reagieren. Bevorzugt setzt man Gele auf Wasserbasis ein, insbesondere Gele auf Wasserbasis mit dispergierten organischen Gelbildnern. Beispielsweise sind solche Gele einsetzbar, wie sie in der deutschen Offenlegungsschrift DE-OS 38 36 138 beschrieben sind. Hydrophile organische Gele auf Basis von modifizierter Fettalkoholalkoxylate sowie von synthetisch erzeugten Polymeren, vorzugsweise Gele auf Basis von Polyvinylalkohol oder Polyacrylsäurederivate, sind besonders bevorzugt.

Bevorzugte handelsübliche Verdicker sind die Rohstoffe Optiflo® H 600/E der Firma SÜD-CHEMIE und PLURIOL A 5000 T 85 der Firma BASF. Optiflo H 600/E ist ein nichtionisches, hydrophob modifiziertes Polymer und PLURIOL A 5000 T 85 ist ein modifiziertes Fettalkoholethoxylat

Es kommen aber auch andere Verdicker, wie Carboxymethylcellulose, nichtionisch und kationisch modifizierte Polyacrylate in Frage.

Die Zubereitung kann noch übliche Zusatzstoffe enthalten wie farbgebende Komponenten, Duftstoffe, etc..

Zur Herstellung der erfindungsgemässen Desinfektionsmittel vermischt man die Bestandteile miteinander. So kann man das Lösungsmittel, vorzugsweise Wasser, die Wirkstoff-Komponenten sowie Verdickungsmittel, Thixotropiermittel, Gelbildner, bzw. Filmbildner, gegebenenfalls Tenside, insbesondere schaumarme, und andere Hilfsstoffe, wie beispielsweise Entschäumer, Korrosionsinhibitoren und Komplexbildner, miteinander vermischen.

Das erfindungsgemäße anwendungsfertige Desinfektionsmittel liegt vorzugsweise bei einem pH-Wert von 8 bis 12, besonders bevorzugt 9 bis 11 vor.

Außerdem ist es bevorzugt, wenn das erfindungsgemäße Desinfektionsmittel zusätzlich ein oder mehrere Komponenten enthält, die unterschiedlich zu den bisher genannten und ausgewählt sind aus den Gruppen der tertiären Aminoxide, anionischen Tenside, Ethersulfate, Alkylpolyglycoside, Kationtenside, Niotensiden, Amphotenside, Silikonentschäumer, Cumolsulfonat, Xylolsulfonat, Toluolsulfonat, hydrophob modifiziertes Polymer, Verdünnungsmittel, Chelatkomplexbildner, Enzyme, Konservierungsmittel, Sequestrierungsmittel, Oxidations-(Bleich)mittel, Farbstoffe und/oder Parfüme.

Als Tenside kommen besonders vorrangig schaumarme Niotenside, wie Fettalkoholalkoxylate mit einer 8 bis 22 C-Atome enthaltenden Alkylgruppe mit oder ohne Verschluß der Endgruppe oder die bekannten verdickenden Aminoxide, zum Einsatz. Wenn Aniontenside zum Einsatz kommen, dann werden sie aufgrund des Schaumverhaltens und der Verträglichkeit mit kationischen Wirkstoffen und/oder Glucoprotamin nur in so geringen Mengen eingesetzt, daß sie bei der Anwendung nicht zu Reklamationen führen.

Ein weiterer Gegenstand der vorliegenden Erfindung ist ein Verfahren zur Pflege einer ärztlichen oder zahnärztlichen Absauganlage, insbesondere von derem Saugschlauch, bei dem in den Saugbereich ein erfindungsgemäßes Desinfektionsmittel eingeführt wird.

Außerdem ist Gegenstand der vorliegenden Erfindung die Verwendung eines erfindungsgemäßen Desinfektionsmittels zur Abtötung von Bakterien, insbesondere Mycobakterien und/oder Pilzen in ärztlichen oder zahnärztlichen Absauganlagen, insbesondere in derem Saugschlauch sowie in der Speischale.

Die folgenden Beispiele sollen die Erfindung weiter erläutern, ohne sie in ihrem Umfang einzuschränken.

### Beispiele

### Beispiel 1

Herstellung eines erfindungsgemäßen Desinfektions-Gels
a) 18 g Carboxymethylcellulose wurden in 950 g Wasser eingestreut, mit 1,0 g einer 50 Gew.-%ige Kaliumhydroxid-Lösung versetzt und unter Rühren vermischt.
b) Nach Abschluß des Quellungsprozesses der Carboxymethylcellulose (2 h - 5 h) wurden der unter a) hergestellten Lösung 8 g einer 50 Gew.-%igen Glucoprotamin®-Lösung, 5 g einer 70 Gew.-%igen Lösung von Dioctyl-dimethylammoniumchlorid in einem Wasser-/Isopropanol-Gemisch im Verhältnis 2 : 3 und 5 g eines C₁₂₋₁₈-Fettalkohol-EO-BuO-Adduktes mit 8 EO und 8 BuO zugesetzt und intensiv vermischt.

### Beispiel 2

Anwendung des im Beispiel 1 hergestellten Gels.

Edelstahlkeimträger gem. DIN 10510 wurden mit dem Testorganismus Enterococcus faecium in Bouillon mit einer zusätzlichen organischen Belastung durch 1 % Mucin und 20 % Blut kontaminiert und 2 h bei Raumtemperatur angetrocknet. Diese Keimträger wurden im Spraynebelschlauch einer zahnärztlichen Absauganlage befestigt und zwar je ein Keimträger am Anfang und am Ende des Schlauches. Zur Desinfektion wurde der Schlauch durch Ansaugen von 200 ml Wasser befeuchtet; danach wurden 15 ml eines Gels gemäß Beispiel 1, dann weitere 100 ml Wasser und danach nochmals 15 ml eines Gels gemäß Beispiel 1 eingesaugt.

Nach 60 Minuten Einwirkzeit erfolgte das Ausspülen des Gels durch Ansaugen von 500 ml Wasser.

Anschließend wurden die Keimträger dem Schlauch entnommen, jeweils in 10 ml Boullion ausgeschüttelt und die Koloniezahl dieser Boullion durch Ausplattieren auf Agarplatten ermittelt.

In entsprechenden Vergleichsversuchen wurde statt dem erfindungsgemäßem Gel
a) eine wäßrige Lösung mit gleicher Konzentration an gleichen antimikrobiellen Wirkstoffen, die
b) jedoch ohne die Eigenschaft der verdickenden Konsistenz in gleichen Mengen, bezogen auf die Konzentration der antimikrobiellen Wirkstoffe, verwendet.

Die Differenz der Logarithmen (Basis 10) der ermittelten Koloniezahlen wurden als Ergebnis (logarithmischer Reduktionsfaktor, log-Rf) ermittelt. Die Ergebnisse sind in der folgenden Tabelle zusammengefaßt:

| **Desinfektionsmedium** | **log-Rf** |
|---|---|
| Gel gemäß Beispiel 1 | > 5,2 |
| Wäßrige Lösung gemäß Beispiel 1 ohne Carboxymethylcellulose | 3,4 |
| Wasser | 2,5 |

## Patentansprüche

1. Verdicktes, anwendungsfertiges Desinfektionsmittel für ärztliche oder zahnärztliche Absauganlagen, enthaltend einen Wirkstoff ausgewählt aus der Gruppe der Alkylpropylendiamine mit der allgemeinen Formel I 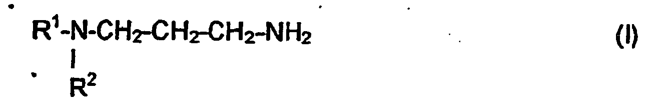 worin R¹ eine Alkyl- oder Alkenylgruppe mit 8 bis 18 Kohlenstoffatomen und R² Wasserstoff, eine Alkylgruppe mit 1 bis 4 Kohlenstoffatomen oder eine Aminoalkylgruppe mit 2 bis 4 Kohlenstoffatomen bedeuten und/oder der Gruppe der als Glucoprotamin® bekannten Produkte, wie sie aus Alkylpropylendiamin der Formel II
R³-NH-CH₂-CH₂-CH₂-NH₂ (II)
in der R³ für eine lineare Alkylgruppe mit 12 bis 14 Kohlenstoffatomen steht, durch Umsetzung mit Verbindungen der Formel III 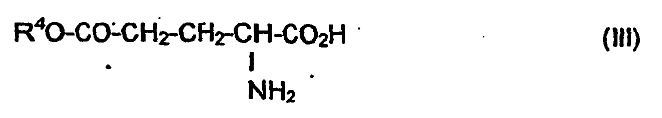 in der R⁴ für Wasserstoff oder eine Akylgruppe mit 1 bis 4 Kohlenstoffatomen steht, im Molverhältnis 1 : 1 bis 1 : 2 bei 60 bis 175°C zugänglich sind, **dadurch gekennzeichnet, daß** das anwendungsfertige Mittel eine Viskosität zwischen 100 und 2000 mPas, vorzugsweise zwischen 350 und 1000 mPas aufweist, gemessen mit einem Brookfield-Digitalviskosimeter, Modell LVTDV-II bei einer Probentemperatur von 20°C unter Verwendung der Spindel Nr. 2(LV-Serie Codierzahl 62) mit einer Spindeldrehung von 12 Umdrehungen/Minute,
wobei der Wert nach 60 Sekunden abgelesen wurde, und als zusätzliche Komponente ein Verdicker enthalten ist.

2. Desinfektionsmittel nach Anspruch 1, **dadurch gekennzeichnet, daß** als Wirkstoff Glucoprotamin®, N,N-Bis(3-aminopropyl)-laurylamin, N-Dodecyl-1,3-propandiamin, N-Kokos-1,3-propandiamin oder ein Gemisch hieraus eingesetzt wird.

3. Desinfektionsmittel nach einem der Ansprüche 1 oder 2, **dadurch gekennzeichnet, daß**, bezogen auf das gesamte Mittel, von 0,01 bis 30 Gew.-%, insbesondere 0,1 bis 15 Gew.-% von einem oder mehreren der genannten Wirkstoffe enthalten sind.

4. Desinfektionsmittel nach einem oder mehreren der Ansprüche 1 bis 3, **dadurch gekennzeichnet, daß** zusätzlich ein zweiter Wirkstoff ausgewählt aus der Gruppe der quartären Ammoniumverbindungen der Formel I 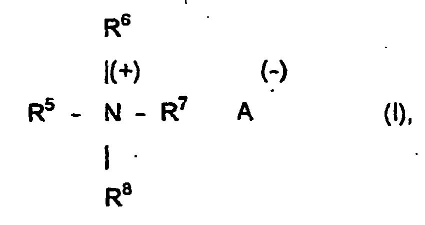 in der R⁵ eine Alkylgruppe mit 6 - 16 C-Atomen, R⁶ eine Alkylgruppe mit 1 - 12 C-Atomen oder eine Benzylgruppe, R⁷ und R⁸ Alkylgruppen mit 1 - 4 C-Atomen oder Hydroxyalkylgruppen mit 2 - 4 C-Atomen und A(-) ein ladungsausgleichendes korrespondierendes Anion bedeutet, enthalten ist.

5. Desinfektionsmittel nach Anspruch 4, **dadurch gekennzeichnet, daß** als zweiter Wirkstoff Benzalkoniumchlorid und/oder Dimethyl-dioctylammoniumchlorid eingesetzt wird.

6. Desinfektionsmittel nach einem der Ansprüche 4 oder 5, **dadurch gekennzeichnet, daß**, bezogen auf das gesamte Mittel von 0,01 bis 30 Gew.-%, insbesondere 0,1 bis 15 Gew.-% des genannten zweiten Wirkstoffes enthalten sind.

7. Desinfektionsmittel nach einem oder mehreren der Ansprüche 1 bis 6, **dadurch gekennzeichnet, daß** das anwendungsfertige Mittel einen pH-Wert von 8 bis 12, vorzugsweise 9 bis 11 aufweist.

8. Desinfektionsmittel nach einem oder mehreren der Ansprüche 1 bis 7, **dadurch gekennzeichnet, daß** zusätzlich ein oder mehrere Komponenten enthalten sind, die unterschiedlich zu den bisher genannten und ausgewählt aus den Gruppen der tertiären Aminoxide, anionischen Tenside, Ethersulfate, Alkylpolyglycoside, Kationtenside, Niotensiden, Amphotenside, Silikonentschäumer, Cumolsulfonat, Xylolsulfonat, Toluolsulfonat, hydrophob modifiziertes Polymer, Verdünnungsmittel, Chelatkomplexbildner, Enzyme, Konservierungsmittel, Sequestrierungsmittel, Oxidations-(Bleich)mittel, Farbstoffe und/oder Parfüme sind.

9. Verfahren zur Pflege einer ärztlichen oder zahnärztlichen Absauganlage, insbesondere deren Saugschlauch, bei dem in den Saugbereich ein Desinfektionsmittel gemäß einem oder mehreren der Ansprüche 1 bis 8 eingeführt wird.

10. Verwendung eines Desinfektionsmittels gemäß einem oder mehreren der Ansprüche 1 bis 8 zur Abtötung von Bakterien und/oder Pilzen in ärztlichen oder zahnärztlichen Absauganlagen, insbesondere in deren Saugschläuchen sowie in der Speischale.

11. Verwendung eines Desinfektionsmittels gemäß einem oder mehreren der Ansprüche 1 bis 8 zur Abtötung von Mycobakterien in ärztlichen oder zahnärztlichen Absaugeinrichtungen, insbesondere in deren Saugschläuchen sowie in der Speischale.

## Claims

1. A thickened, ready-for-use disinfectant for medical or dental suction apparatus, which includes an active substance selected from the group of alkylpropylenediamines of general formula I 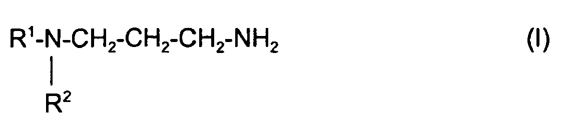 wherein R¹ represents an alkyl or alkenyl group with 8 to 18 carbon atoms and R² represents hydrogen, an alkyl group with 1 to 4 carbon atoms or an aminoalkyl group with 2 to 4 carbon atoms, and/or from the group of products known as Glucoprotamin®, as obtained from an alkylpropylenediamine of formula II
R³-NH-CH₂-CH₂-CH₂-NH₂ (II)
wherein R³ represents a linear alkyl group with 12 to 14 carbon atoms, by reaction with compounds of formula III 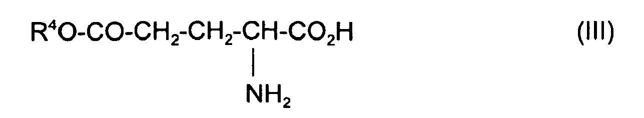 wherein R⁴ represents hydrogen or an alkyl group with 1 to 4 carbon atoms, at a molar ratio of from 1:1 to 1:2 at 60 to 175°C, **characterized in that** the ready-for-use agent has a viscosity between 100 and 2000 mPas, preferably between 350 and 1000 mPas, measured with a Brookfield digital viscometer, model LVTDV-II at a sample temperature of 20°C using a spindle No. 2 (LV series, code number 62) at a spindle rotation of 12 revolutions/minute, the value being read after 60 seconds, and a thickening agent being included as additional component.

2. The disinfectant according to claim 1, **characterized in that** Glucoprotamin®, N,N-bis(3-aminopropyl)laurylamine, N-dodecyl-1,3-propanediamine, N-coco-1,3-propanediamine or a mixture thereof is used as active substance.

3. The disinfectant according to any of claims 1 or 2, **characterized in that** 0.01 to 30 wt.-%, particularly 0.1 to 15 wt.-%, relative to the overall agent, of one or more of the above-mentioned active substances are included.

4. The disinfectant according to one or more of claims 1 to 3, **characterized in that** a second active substance selected from the group of quaternary ammonium compounds of formula I, 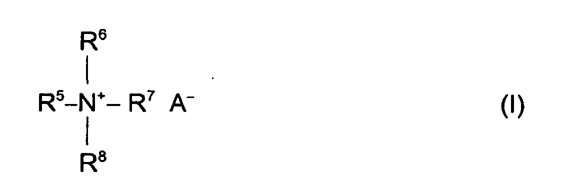 wherein R⁵ represents an alkyl group with 6 to 16 C atoms, R⁶ represents an alkyl group with 1 to 12 C atoms or a benzyl group, R⁷ and R⁸ represent alkyl groups with 1 to 4 C atoms or hydroxyalkyl groups with 2 to 4 C atoms, and A⁻ represents a charge-compensating anion, is included in addition.

5. The disinfectant according to claim 4, **characterized in that** benzalkonium chloride and/or dimethyldioctylammonium chloride is used as second active substance.

6. The disinfectant according to any of claims 4 or 5, **characterized in that** 0.01 to 30 wt.-%, especially 0.1 to 15 wt.-%, relative to the overall agent, of said second active substance is included.

7. The disinfectant according to one or more of claims 1 to 6, **characterized in that** the ready-for-use agent has a pH value of from 8 to 12, preferably from 9 to 11.

8. The disinfectant according to one or more of claims 1 to 7, **characterized in that** one or more components other than those mentioned above are included in addition, selected from the group of tertiary amine oxides, anionic surfactants, ether sulfates, alkylpolyglycosides, cationic surfactants, nonionic surfactants, amphosurfactants, silicone defoamers, cumenesulfonate, xylenesulfonate, toluenesulfonate, hydrophobically modified polymers, diluents, chelating agents, enzymes, preservatives, sequestering agents, oxidizing (bleaching) agents, dyes and/or perfumes.

9. A method for the maintenance of a medical or dental suction apparatus, particularly of the suction tube thereof, in which method a disinfectant according to one or more of claims 1 to 8 is introduced into the suction area.

10. Use of a disinfectant according to one or more of claims 1 to 8 in the destruction of bacteria and/or fungi in medical or dental suction apparatus, particularly in the suction tube thereof and in the spitting cup.

11. Use of a disinfectant according to one or more of claims 1 to 8 in the destruction of mycobacteria in medical or dental suction apparatus, particularly in the suction tube thereof and in the spitting cup.

## Revendications

1. agent désinfectant épaissi, prêt à l'emploi, pour installations d'aspiration médicales ou dentaires, contenant un agent actif choisi dans le groupe des alkylpropylènediamines de formule générale 1 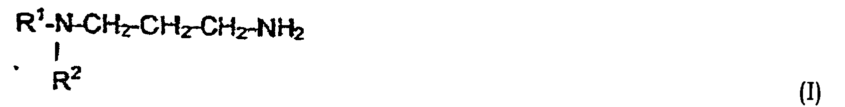 dans laquelle R¹ représente un groupe alkyle ou alcényle comportant 8 à 18 atomes de carbone et R² un atome d'hydrogène, un groupe alkyle comportant 1 à 4 atomes de carbone ou un groupe aminoalkyle comportant 2 à 4 atomes de carbone, et/ou dans le groupe des produits connus en tant que glucoprotamine®, tels que ceux pouvant être obtenus dans un rapport molaire de 1:1 à 1:2 et de 60°C à 175°C,à partir d'alkylpropylènediamine de formule II
**R**^{**3**}**-NH-CH**_{**2**}**-CH**_{**2**}**-CH**_{**2**}**-NH**_{**2**} (II)
dans laquelle R³ représente un groupe alkyle linéaire comportant 12 à 14 atomes de carbone, par conversion avec des composés de formule III 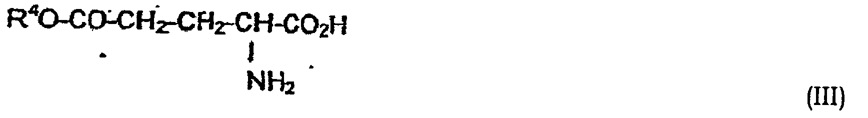 dans laquelle R⁴ représente un atome d'hydrogène ou un groupe alkyle comportant 1 à 4 atomes de carbone,
**caractérisé en ce que**
l'agent prêt à l'emploi a une viscosité comprise entre 100 et 2000 mPas, de préférence entre 350 et 1000 mPas, mesurée avec un viscosimètre numérique de Brookfield, modèle LVTDV-II, à une température des échantillons de 20°C en utilisant la broche No 2 (série LV, indice de codage 62) avec une vitesse de rotation de la broche de 12 rotations par minute, la valeur ayant été relevée au bout de 60 secondes, et il contient un épaississant en tant que composant supplémentaire.

2. agent désinfectant selon la revendication 1,
**caractérisé en ce que**
comme agent actif, on utilise de la glucoprotamine®, de la N,N-bis(3-aminopropyl)-laurylamine, de la N-dodécyl-propane-1,3-diamine, de la N-coco-propane-1,3-diamine ou un mélange de ces substances.

3. agent désinfectant selon l'une quelconque des revendications 1 ou 2,
**caractérisé en ce qu'**
il contient, par rapport au produit total, de 0,01 à 30 % en poids, en particulier de 0,1 à 15 % en poids d'un ou plusieurs des agents actifs indiqués.

4. agent désinfectant selon l'une quelconque ou plusieurs des revendications 1 à 3,
**caractérisé en ce qu'**
il contient en plus, un deuxième agent actif choisi dans le groupe des composés d'ammonium quartenaire de formule I 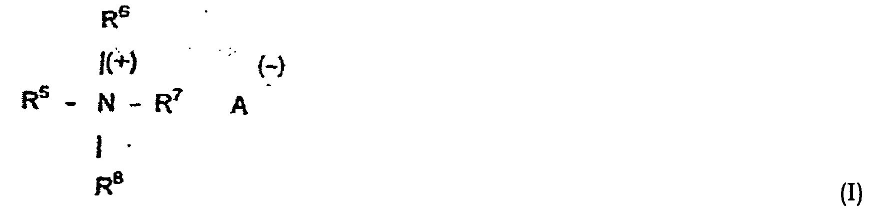 dans laquelle R⁵ représente un groupe alkyle comportant 6 à 16 atomes de C, R⁶ un groupe alkyle comportant 1 à 12 atomes de C ou un groupe benzyle, R⁷ et R⁸ des groupes alkyle comportant 1 à 4 atomes de C ou des groupes hydroxyalkyle comportant 2 à 4 atomes de C et A(-) un anion correspondant compensateur de charge.

5. agent désinfectant selon la revendication 4,
**caractérisé en ce que**
comme deuxième agent actif, on utilise du chlorure de benzalkonium et/ou de chlorure de diméthyl-dioctylammonium.

6. agent désinfectant selon l'une quelconque des revendications 4 ou 5,
**caractérisé en ce qu'**
il contient, par rapport à produit total, de 0,01 à 30 % en poids, en particulier de 0,1 à 15 % en poids du deuxième agent actif indiqué.

7. agent désinfectant selon l'une quelconque ou plusieurs des revendications 1 à 6,
**caractérisé en ce que**
le produit prêt à l'emploi présente un pH de 8 à 12, de préférence de 9 à 11.

8. agent désinfectant selon l'une quelconque ou plusieurs des revendications 1 à 7,
**caractérisé en ce qu'**
il contient en plus un ou plusieurs composants qui sont différents de ceux indiqués jusqu'ici et sont choisis dans les groupes des aminoxydes tertiaires, des tensioactifs anioniques, des éthersulfates, des alkylpolyglycosides, des tensioactifs cationiques, des tensioactifs non ioniques, des tensioactifs amphotères, des agents antimousse à base de silicone, du sulfonate de cumène, du sulfonate de xylène, du sulfonate de toluène, du polymère modifié par voie hydrophobe, des agents de dilution, des agents complexants chélatants, des enzymes, des agents de conservation, des agents de séquestration, des agents (de blanchiment) oxydants, des colorants et/ou des parfums.

9. Procédé d'entretien d'une installation d'aspiration médicale ou dentaire, en particulier de son tuyau d'aspiration, selon lequel un agent désinfectant selon l'une quelconque ou plusieurs des revendications 1 à 8 est introduit dans la zone d'aspiration.

10. Utilisation d'un agent désinfectant selon l'une quelconque ou plusieurs des revendications 1 à 8, pour tuer les bactéries et/ou les champignons dans des installations médicales ou dentaires, en particulier dans leurs tuyaux d'aspiration ainsi que dans les crachoirs.

11. Utilisation d'un agent désinfectant selon l'une quelconque ou plusieurs des revendications 1 à 8, pour tuer des mycobactéries dans des dispositifs d'aspiration médicaux ou dentaires, en particulier dans leurs tuyaux d'aspiration ainsi que dans les crachoirs.
